# EUROPEAN PATENT SPECIFICATION

(11) **EP 3 928 720 B1**
(45) Date of publication and mention of the grant of the patent: **22.06.2022**
(21) Application number: 20217077.5
(22) Date of filing: 23.12.2020
(51) Int. Cl.: A61B 17/15, A61B 17/56

(54) **DISTAL FEMORAL CONDYLE BONE DEFECT OSTEOTOMY GUIDE**
KNOCHENDEFEKTOSTEOTOMIELEHRE FÜR DISTALEN FEMURKONDYLUS
GABARIT D'OSTÉOTOMIE EN CAS DE DÉFAUTS OSSEUX DU CONDYLE DISTAL FÉMORAL

(30) Priority: 28.06.2020 CN 202010599163
(43) Date of publication of application: 29.12.2021
(73) Proprietor: Beijing Lidakang Technology Co., Ltd, Beijing 101399 (CN)
(72) Inventor: LI, Jiandong, Zhaoquanying, beijing (CN); JIA, Jingliang, Zhaoquanying, beijing (CN); WANG, Donglin, Zhaoquanying, beijing (CN)
(74) Representative: Petculescu, Ana-Maria

(56) References cited:
- CN-A- 107 049 415
- US-A- 6 013 081
- US-A1- 2009 125 114
- US-A1- 2019 231 365

## Description

### CROSS-REFERENCE TO RELATED APPLICATIONS

This application is based upon and claims priority to Chinese Patent Application No. 202010599163.1, filed on June 28, 2020.

### TECHNICAL FIELD

The present invention relates to the technical field of medical instruments, in particular to a distal femoral condyle bone defect osteotomy positioner used for total knee arthroplasty.

### BACKGROUND

Knee joint is the largest and most complex joint in human body. Knee arthroplasty is an operation to replace all or part of the damaged natural knee joint with knee prosthesis. Cash knee arthroplasty has developed into a mature orthopedic operation. In total knee arthroplasty, a multi-plane osteotomy is needed on the femoral side to install the femoral side prosthesis.

Generally, it is necessary to perform osteotomy at the distal femur first, and then measure the size of the femoral condyle with this osteotomy plane as the reference plane, and then determine the position of osteotomy module. The osteotomy locator for distal femoral condyle bone defect is mainly used to determine the valgus angle of the distal femur and then determine the amount of bone defect inside and outside the distal femur. And 5mm or 10mm correction is made to select the proper size of distal femur filling prosthesis. Generally, the position of the osteotomy module is determined by reference of the femoral medullary cavity, and the valgus angle and anteroposterior position of the osteotomy module are determined, or the osteotomy position of the osteotomy module is determined by reference of the distal femur bone surface.

In the prior art, distal femoral condyle bone defect osteotomy positioners are generally divided into left and right positioners, and the left knee joint uses the left positioners and the right knee joint uses the right positioners, which is complicated in operation and high in equipment cost. Generally, only a limited fixed valgus angle can be provided, such as 5° or 6°. The fixed valgus angle is difficult to meet the personalized choice of the existing population, which causes great operational limitations for the surgical operation. In addition, there is no better way to correct the bone defect in the distal femur, which depends entirely on the experience of doctors. If the doctors make wrong judgment and operation, it will cause the imbalance of filling prosthesis in the distal femur, which will directly affect the patient's physiological force line and cause an operation failure.

US 2019/231365 A1 discloses a distal femoral condyle bone defect positioner according to the preamble of claim 1.

### SUMMARY

The distal femoral condyle bone defect osteotomy positioner is simple in structure, simple and convenient to operate, and can be shared by left and right sides; it is adjustable in valgus angles of 0°, 3°, 5°, 7° and 9° with accurate correction of N0, 5mm and 10mm for distal femur bone defects.

To solve the above technical problems, the embodiment of the present invention provides the following solution: a distal femoral condyle bone defect osteotomy positioner, characterized in comprising a distal femur bone defect osteotomy module, a sliding locking positioning module and a distal femur valgus angle positioning module, wherein a front side of the bottom of the distal femur bone defect osteotomy module is provided with an installation surface matching femoral anterior condyle, and a rear side of the top of the distal femur bone defect osteotomy module is provided with three correction osteotomy grooves which are arranged in parallel in the anteroposterior direction;
the sliding locking positioning module is connected behind the distal femur bone defect osteotomy module and comprises a sliding locking positioning module body and a press-type locking hook, a middle part of the pressing locking hook is rotationally connected with the top of the sliding locking positioning module body, a spring is arranged between a lower part of the rear end of the press-type locking hook and the sliding locking positioning module body, and the front end of the press-type locking hook is a hook which can be detachably hooked with the correction osteotomy grooves;
the distal femur valgus angle positioning module is arranged under the sliding locking positioning module, including a distal femur valgus angle positioning module body, a valgus angle positioning sleeve, a valgus angle dial and a valgus angle toggle lever; the front end of the valgus angle toggle lever is inserted into and fixed with the distal femur valgus angle positioning module body, and the rear end of the valgus angle toggle lever extends out from the rear of the distal femur valgus angle positioning module body and is sleeved with the valgus angle positioning sleeve, and the rear part of the valgus angle positioning sleeve is limited by a fixed nut screwed with the valgus angle toggle lever;
the valgus angle dial is arranged above the valgus angle dial toggle lever and is rotationally connected with the distal femur valgus angle positioning module body, the rear end of the valgus angle dial is an arc edge, the center of the arc edge is a 0° dial point, the axis of a fastening screw, the axis of a rotating shaft and the 0°dial point are positioned on a same vertical surface, a 3° dial point, a 5° dial point, a 7° dial point and a 9°dial point on the L side are arranged in sequence from the 0° dial point to the left, anda 3° dial point, a 5° dial point, a 7° dial point and a 9°dial point on the R side are arranged in sequence to the right;
the arc edge is provided with a dial groove respectively corresponding to the 0°dial point, the 3° dial point, the 5° dial point, the 7° dial point and the 9°dial point on the L side and the 0°dial point, the 3° dial point, the 5° dial point, the 7° dial point and the 9°dial point on the R side, and the front end of the valgus angle positioning sleeve is provided with a convex positioning tip which is inserted into the dial groove.

Wherein, the sliding locking positioning module also comprises two parallel optical axes, the left end and the right end of a bottom plate of the sliding locking positioning module body are respectively connected with one of the parallel optical axis, the left side and the right side of the distal femur valgus angle positioning module body are respectively provided with a positioning sliding hole, and the two parallel optical axes are correspondingly inserted into the two positioning sliding holes.

Wherein, the left side and the right side of the top of the distal femur bone defect osteotomy module are both provided with oblique nail holes for fixation, and the front side of the top of the distal femur bone defect osteotomy module is provided with nail holes with adjustable positions.

Wherein, a connecting hole is arranged on the rear end surface of the distal femur bone defect osteotomy module, a connecting rod is arranged on the front end surface of the sliding locking positioning module body, and the connecting rod is inserted into the connecting hole.

Wherein, the top part of the sliding locking positioning module body is provided with two lateral support plates, a spring mounting hole is arranged on a connecting surface between the two lateral support plates, a spring inserted with the spring mounting hole is arranged below the rear end of the press-type locking hook, the middle part of the press-type locking hook is supported and positioned between the two lateral support plates through a support shaft, and the front end of the press- type locking hook is a hook which is detachably hooked with the correction osteotomy groove.

Wherein, the distal femur valgusangle positioning module body comprises an upper side plate and a lower side plate, a toggle hole is arranged between the upper side plate and the lower side plate and the front end, the front end of the valgus angle toggle lever is inserted into the toggle hole and fixedly connected with the upper side plate through a fastening screw, the valgus angle dial is arranged between the valgus angle toggle lever and the upper side plate, a rotating shaft is arranged at the rear end on the central line of the upper side plate, and the front end of the valgus angle dial is rotationally connected with the upper side plate through the rotating shaft.

Wherein, the correction dimensions of the three correction osteotomy grooves are 0, 5mm and 10mm respectively.

Wherein, the press-type locking hook is provide with a plurality of convex edges.

Wherein, the lower end of one of the parallel optical axes is provided with an anti-separation mechanism.

Wherein, one of the two positioning sliding holes is a circular hole, and the other one is an elliptical hole.

The above solution of the present invention at least includes the following beneficial effects:
According to the invention, the functions of correcting osteotomy for medial and lateral bone defect of distal femoral condyle of the left and right legs and adjusting and positioning the valgus angle of the left and right legs are combined into one, and a continuous valgus angle of 0-9°can be selected and adjusted, so that the positioning of the valgus angle of osteotomy of the distal femur is greatly facilitated for doctors, and the medial and lateral bone defect of the distal femur is accurately corrected by 5mm or 10mm, so that the operation efficiency is improved and the operation is simplified, thereby facilitating use by the doctors as much as possible and ensuring the postoperative safety of patients.

### BRIEF DESCRIPTION OF DRAWINGS

FIG. 1 is a schematic structural diagram of the present invention;
FIG. 2 is a front view of the present invention;
FIG. 3 is a left side view of the present invention;
FIG. 4 is a top view of the present invention;
FIG. 5 is an enlarged view of an exploded view of the structure of the distal femur bone defect osteotomy module in the present invention;
FIG. 6 is an enlarged view of an exploded view of the structure of the sliding locking positioning module in the present invention;
FIG. 7 is an enlarged view of the exploded view of the distal femur valgus angle positioning module of the present invention.

### Description of reference signs:

1. distal femur bone defect osteotomy module; 11. Installation surface; 12. Oblique nail hole; 13, position-adjustable nail hole; 14. Correction osteotomy groove; 15. Connecting hole; 2. Sliding locking positioning module; 21. Sliding locking positioning module body; 22. Press-type locking hook; 221, Hook; 23, Parallel optical axis; 24. Connecting rod; 25. Lateral support plate; 26. Spring mounting hole; 27. Spring; 3. Distal femur valgus angle positioning module; 31. Distal femur valgus angle positioning module body; 32, Valgus angle positioning sleeve; 321. Convex positioning tip; 33, Valgus angle dial; 331. Arc edge; 332, Dial groove; 34. Valgus angle toggle lever; 35. Positioning sliding hole; 36, Toggle hole; 37, Fastening screw; 38, Rotation shaft.

### DETAILED DESCRIPTION OF THE EMBODIMENTS

Exemplary embodiments of the present disclosure will be described in more detail below with reference to the accompanying drawings. Although exemplary embodiments of the disclosure are shown in the drawings, it should be understood that the disclosure may be embodied in various forms and should not be limited by the embodiments set forth herein. On the contrary, these embodiments are provided to enable a more thorough understanding of the disclosure and to fully convey the scope of the disclosure to those skilled in the art.

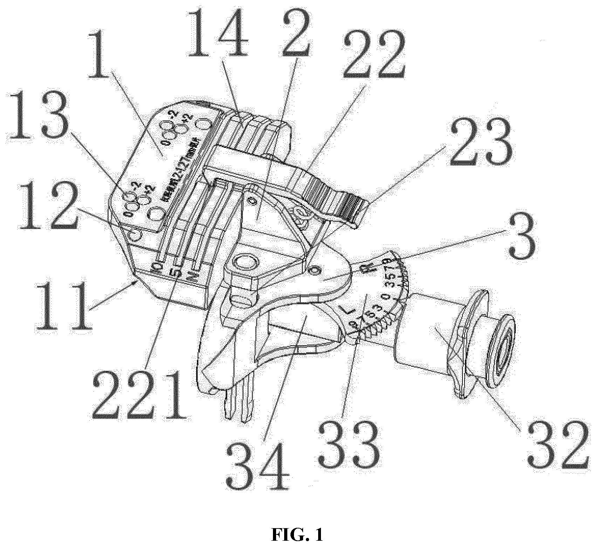

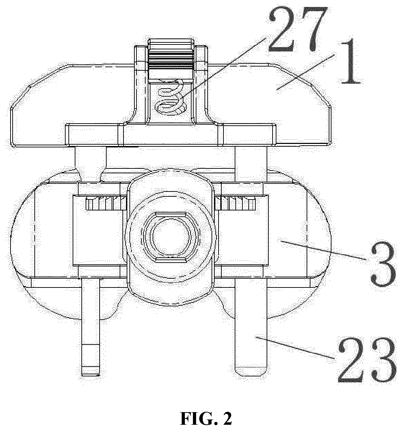

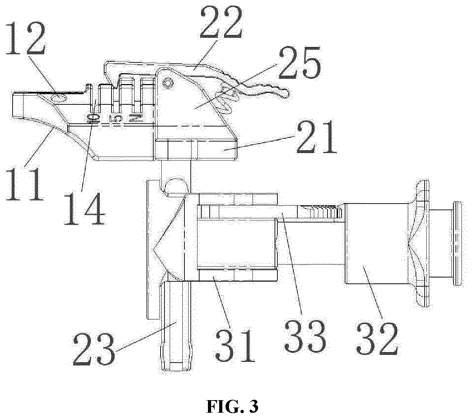

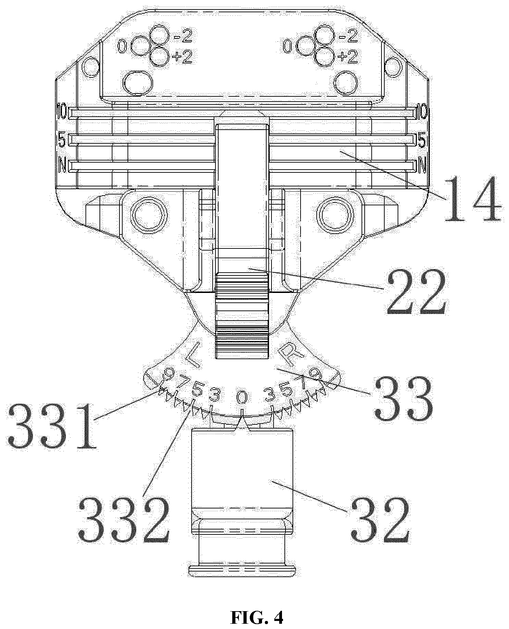

As shown in FIGS. 1-4, the present invention provides a distal femoral condyle bone defect osteotomy positioner, which includes a distal femur bone defect osteotomy module 1, a sliding locking positioning module 2 and a distal femur valgus angle positioning module 3, wherein the sliding locking positioning module 2 is arranged behind the distal femur bone defect osteotomy module 1 and the distal femur valgus angle positioning module 3 is arranged below the sliding locking positioning module 2.

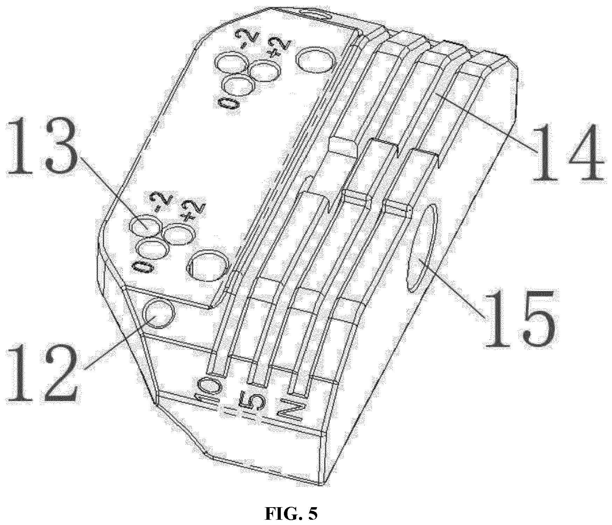

As shown in FIG. 5, the front side of the bottom of the distal femurl bone defect osteotomy module 1 is provided with an installation surface 11 which is attached to the anterior condyle of the femur, so as to ensure the stability of osteotomy. The front left and right sides of the top of the distal femur bone defect osteotomy module 1 are provided with oblique nail holes 12 for fixing, which are accurately matched with fixing nails to ensure the stability of the osteotomy module. The front side of the top of the distal femur bone defect osteotomy module 1 is provided with a position-adjustable nail hole 13, and the rear side of the top is provided with three correction osteotomy grooves 14 arranged in parallel, and the rear end face of the distal femur bone defect osteotomy module 1 is provided with a connecting hole 15. In this embodiment, the correction dimensions of the three correction osteotomy grooves 14 are 0, 5mm and 10mm, respectively, so as to ensure the accuracy of distal femur correction. A nail hole with an adjustable position is designed on the osteotomy module 1 for bone defect at the distal femur, and when doctors need to increase or decrease the amount of bone cut, the purpose of adjusting the amount of osteotomy can be achieved by adjusting the position of the nail hole.

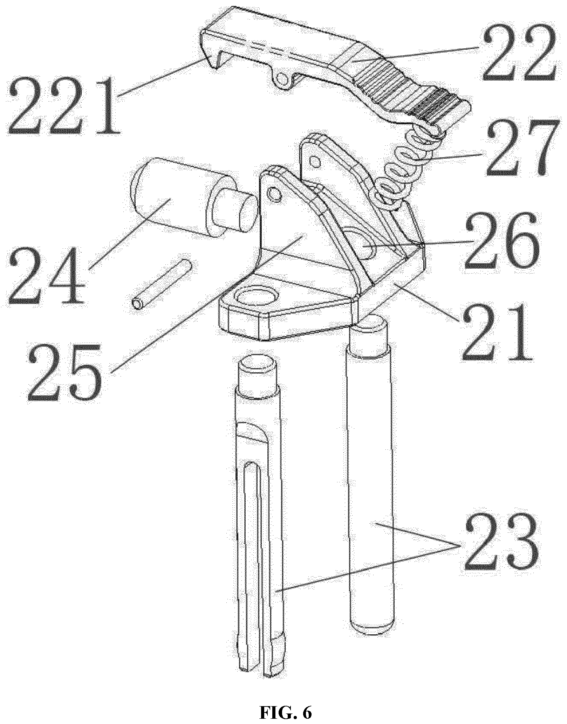

As shown in FIG. 6, the sliding locking and positioning module 2 includes a sliding locking and positioning module body 21, a press-type locking hook 22 and a parallel optical axis 23. The front end face of the sliding locking and positioning module body 21 is provided with a connecting rod 24 which is inserted into the connecting hole 15. The top of the sliding locking and positioning module body 21 is provided with two lateral support plates 25, and the connecting surface between the two lateral support plates 25 is provided with a spring mounting hole 26. A spring 27 inserted into the spring mounting hole 26 is provided below the rear end of the press-type locking hook 22.

The middle part of the press-type locking hook 22 is supported and positioned between the two lateral support plates 25 through a supporting shaft, and the front end of the press-type locking hook 22 is a hook 221 which can be detachably hooked with the correction osteotomy groove 14, and a plurality of ribs are arranged on the press-type locking hook 22, so that the friction force is increased, and doctors feel better when pressing. The press-type locking hook 22 ensures the safety and smoothness of mounting and dismounting the osteotomy module.

The left and right ends of the bottom plate of the sliding locking positioning module body 21 are respectively connected with a parallel optical axis 23, so as to ensure that they can slide up and down freely, and the parallel optical axis on one side is designed with an anti-disengagement mechanism, so as to ensure the safety in the transmission process of the instrument during operation.

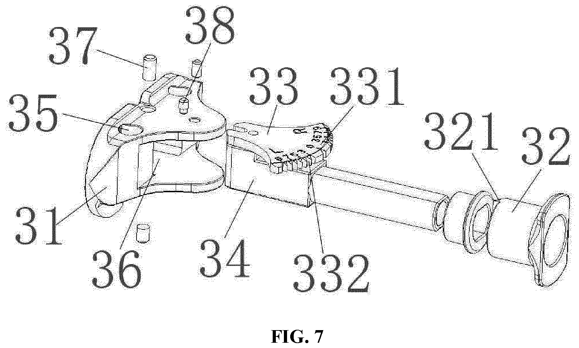

As shown in FIG. 7, the distal femur valgus angle positioning module 3 includes a distal femur valgus angle positioning module body 31, an valgus angle positioning sleeve 32, an valgus angle dial 33 and an valgus angle toggle lever 34. The left and right sides of the distal femurl valgus angle positioning module body 31 are respectively provided with a positioning sliding hole 35. The two parallel optical axes 23 are correspondingly inserted into two positioning sliding holes 35, one of which is a circular hole and the other is an elliptical hole, thus effectively avoiding stalling caused by accumulated errors of parallel optical axes when sliding up and down.

The distal femur valgus angle positioning module body 31 includes upper and lower lateral plates, and a toggle hole 36 is formed between the upper and lower lateral plates to the front ends. The front end of the valgus angle toggle rod 34 is inserted into the toggle hole 36 and fixedly connected with the upper lateral plate by a fastening screw 37. The rear end of the valgus angle toggle lever 34 protrudes from the rear of the distal femur valgus angle positioning module body 31 and then is sleeved with an valgus angle positioning sleeve 32, and the rear end of the valgus angle positioning sleeve 32 passes through the valgus angle positioning sleeve 32 The valgus angle dial 33 is arranged between the valgus angle toggle lever 34 and the upper lateral plate, the rear end of the center line of the upper side plate is provided with a rotating shaft 38, the front end of the eversion angle dial 33 is rotationally connected with the upper lateral plate through the rotating shaft 38, the rear end of the valgus angle dial 33 is an arc edge 331, and the center of the arc edge 331 is a 0° dial point. The axis of the fastening screw 37, the axis of the rotating shaft 38 and the 0° dial point are located on the same vertical plane, and the dial points on the arc edge 331 are 3°, 5°, 7° and 9° on the L side from the 0° dial point to the left and 3°, 5°, 7° and 9° on the R side to the right.

The arc edge 331 corresponds to 0° dial points, and dial grooves 332 are respectively arranged at 3°, 5°, 7° and 9° dial points on the L side and 3°, 5°, 7° and 9° dial points on the R side. The front end of the valgus angle positioning sleeve 32 is provided with a convex positioning tip 321, which is inserted into the dial groove 332 The front side of the valgus angle positioning sleeve is designed with a convex positioning tip, and after the valgus angle adjustment is completed, the convex positioning tip can be embedded into the dial groove, thus ensuring that the valgus angle will not be changed by the vibration of osteotomy during operation.

According to the present invention, the functions of correcting osteotomy for medial and lateral bone defect of distal femoral condyleof the left and right legs and adjusting and positioning the valgus angle of the left and right legs are combined into one, and a continuous valgus angle of 0-9°can be selected and adjusted, so that the positioning of the valgus angle of osteotomy of the distal femur is greatly facilitated for doctors, and the medial and lateral bone defect of the distal femur is accurately corrected by 5mm or 10mm, so that the operation efficiency is improved and the operation is simplified, thereby facilitating use by the doctors as much as possible and ensuring the postoperative safety of patients.

The above is the preferred embodiment of the present invention, and it should be pointed out that for ordinary people in the technical field, without departing from the principle of the present invention, several improvements and embellishments can be made.

## Claims

1. A distal femoral condyle bone defect osteotomy positioner, comprising a distal femur bone defect osteotomy module (1), a sliding locking positioning module (2) and a distal femur valgus angle positioning module (3), wherein a front side of the bottom of the distal femur bone defect osteotomy module is provided with an installation surface (11) matching femoral anterior condyle;
the sliding locking positioning module is connected behind the distal femur bone defect osteotomy module and comprises a sliding locking positioning module body (21)
the distal femur valgus angle positioning module is arranged under the sliding locking positioning module, including a distal femur valgus angle positioning module body (31), a valgus angle positioning sleeve (32), a valgus angle dial and a valgus angle toggle lever (34);
; the front end of the valgus angle toggle lever is inserted into and fixed with the distal femur valgus angle positioning module body, and the rear end of the valgus angle toggle lever extends out from the rear of the distal femur valgus angle positioning module body and is sleeved with the valgus angle positioning sleeve, and the rear part of the valgus angle positioning sleeve is limited by a fixed nut screwed with the valgus angle toggle lever;
the valgus angle dial is arranged above the valgus angle dialtoggle lever and is rotationally connected with the distal femur valgus angle positioning module body, the rear end of the valgus angle dial is an arc edge, the center of the arc edge is a 0° dial point, the axis of a fastening screw, the axis of a rotating shaft and the 0° dial point are positioned on a same vertical surface, a 3° dial point, a 5° dial point, a 7° dial point and a 9° dial point on the L side are arranged in sequence from the 0° dial point to the left, and a 3° dial point, a 5° dial point, a 7° dial point and a 9° dial point on the R side are arranged in sequence to the right;
the arc edge is provided with a dial groove respectively corresponding to the 0° dial point, the 3° dial point, the 5° dial point, the 7° dial point and the 9° dial point on the L side and the 0° dial point, the 3° dial point, the 5° dial point, the 7° dial point and the 9° dial point on the R side, and the front end of the valgus angle positioning sleeve is provided with a convex positioning tip (321) which is inserted into the dial groove; **characterised in that** a rear side of the top of the distal femur bone defect osteotomy module (1) is provided with three correction osteotomy grooves (14) which are arranged in parallel in the anteroposterior direction; and **in that**
the sliding locking positioning module comprises a press-type locking hook (22), a middle part of the press-type locking hook is rotationally connected with the top of the sliding locking positioning module body (21), a spring (27) is arranged between a lower part of the rear end of the press-type locking hook and the sliding locking positioning module body, and the front end of the press-type locking hook is a hook (221) which can be detachably hooked with the correction osteotomy grooves.

2. The distal femoral condyle bone defect osteotomy positioner according to claim 1, **characterized in that** the sliding locking positioning module also comprises two parallel optical axes (23), the left end and the right end of a bottom plate of the sliding locking positioning module body are respectively connected with one of the parallel optical axis, the left side and the right side of the distal femur valgus angle positioning module body are respectively provided with a positioning sliding hole (35), and the two parallel optical axes are correspondingly inserted into the two positioning sliding holes.

3. The distal femoral condyle bone defect osteotomy positioner according to claim 1, **characterized in that** the left side and the right side of the top of the distal femur bone defect osteotomy module are both provided with oblique nail holes (12) for fixation, and the front side of the top of the distal femur bone defect osteotomy module is provided with nail holes (13) with adjustable positions.

4. The distal femoral condyle bone defect osteotomy positioner according to claim 1, **characterized in that** a connecting hole (15) is arranged on the rear end surface of the distal femur bone defect osteotomy module, a connecting rod (24) is arranged on the front end surface of the sliding locking positioning module body, and the connecting rod is inserted into the connecting hole.

5. The distal femoral condyle bone defect osteotomy positioner according to claim 1, **characterized in that** the top part of the sliding locking positioning module body is provided with two lateral support plate; (25), a spring mounting hole (26) is arranged on a connecting surface between the two lateral support plates, a spring (27) inserted with the spring mounting hole is arranged below the rear end of the press-type locking hook, the middle part of the press-type locking hook is supported and positioned between the two lateral support plates through a support shaft, and the front end of the press- type locking hook is a hook which is detachably hooked with the correction osteotomy groove.

6. The distal femoral condyle bone defect osteotomy positioner according to claim 1, **characterized in that** the distal femur valgus angle positioning module body comprises an upper side plate and a lower side plate, a toggle hole (36) is arranged between the upper side plate and the lower side plate and the front end, the front end of the valgus angle toggle lever is inserted into the toggle hole and fixedly connected with the upper side plate through a fastening screw, the valgus angle dial is arranged between the valgus angle toggle lever and the upper side plate, a rotating shaft (38) is arranged at the rear end on the central line of the upper side plate, and the front end of the valgus angle dial is rotationally connected with the upper side plate through the rotating shaft.

7. The distal femoral condyle bone defect osteotomy positioner according to claim 1, **characterized in that** the correction dimensions of the three correction osteotomy grooves are 0, 5mm and 10mm respectively.

8. The distal femoral condyle bone defect osteotomy positioner according to claim 1, **characterized in that** the press-type locking hook is provided with a plurality of convex edges.

9. The distal femoral condyle bone defect osteotomy positioner according to claim 2, **characterized in that** the lower end of one of the parallel optical axes is provided with an anti-separation mechanism.

10. The distal femoral condyle bone defect osteotomy positioner according to claim 2, **characterized in that** one of the two positioning sliding holes is a circular hole, and the other one is an elliptical hole.

## Patentansprüche

1. Positionierungsvorrichtung für die Osteotomie eines distalen Femurkondylusknochendefekts, **dadurch gekennzeichnet, dass** sie ein distales Femurknochendefekt-Osteotomiemodul, ein verschiebbare verriegelnde Positionierungsmodul und ein distales Femur-Valguswinkel-Positionierungsmodul umfasst, wobei eine Vorderseite der Boden des distalen Femurknochendefekt-Osteotomiemoduls mit einer Installationsfläche versehen ist, die mit dem vorderen Femurkondylus übereinstimmt, und eine Rückseite der Oberseite des distalen Femurknochendefekt-Osteotomiemoduls mit drei Korrektur-Osteotomienuten versehen ist, die parallel in anteroposteriorer Richtung angeordnet sind;
wobei das verschiebbare verriegelnde Positionierungsmodul hinte r dem distalen Femurknochendefekt-Osteotomiemodul angeordnet ist und einen verschiebbaren verriegelnden Positionierungsmodulkörper und einen pressenden verriegelnden Haken umfasst, wobei ein mittlerer Teil des pressenden verriegelnden Hakens drehbar mit der Oberseite des verschiebbaren verriegelnden Positionierungsmodulkörpers verbunden ist, wobei eine Feder zwischen einem unteren Teil des hinteren Endes des pressenden verriegelnden Hakens und dem verschiebbaren verriegelnden Positionierungsmodulkörper angeordnet ist und das vordere Ende des pressenden verriegelnden Hakens ein Haken ist, der lösbar mit den Korrektur-Osteotomienuten eingehakt werden kann;
wobei das distales Femur-Valguswinkel-Positionierungsmodul unter verschiebbaren verriegelnden Positionierungsmodulkörper angeordnet ist und einen distales Femur-Valguswinkel-Positionierungsmodulkörper, eine Valguswinkel-Positionierungshülse, eine Valguswinkel-Einstellscheibe und einen Valguswinkel-Kipphebel umfasst; wobei das vordere Ende des Valguswinkel-Kipphebels in den distalen Femur-Valguswinkel-Positionierungsmodulkörper eingeführt ist und daran befestigt, und wobei das hintere Ende des Valguswinkel-Kipphebels sich von der Rückseite des distalen Femur-Valguswinkel-Positionierungsmodulkörpers erstreckt und m it der Valguswinkel-Positionierungshülse ummantelt ist, wobei der hintere Teil der Valguswinkel-Positionierungshülse durch eine feststehende Mutter begrenzt ist, die mit dem Valguswinkel-Kipphebel verschraubt ist;
wobei die Valguswinkel-Einstellscheibe oberhalb des Valguswinkel-Kipphebels angeordnet ist und drehbar mit dem distalen Femur-Valguswinkel-Positionierungsmodulkörpers verbunden ist, wobei das hintere Ende der Alguswinkel-Einstellscheibe eine Bogenkante ist, wobei die Mitte der Bogenkante ein Skalenpunkt (dial point) von 0° ist, wobei die Achse einer Befestigungsschraube, die Achse einer rotierenden Welle und der Skalenpunkt von 0° auf derselben vertikalen Fläche positioniert sind, wobei ein Skalenpunkt von 3°, ein Skalenpunkt von 5°,ein Skalenpunkt von 7° und ein Skalenpunkt von 9° auf der L-Seite nacheinander vom Skalenpunkt von 0° nach links angeordnet sind, und wobei ein Skalenpunkt von 3°, ein Skalenpunkt von 5°, ein Skalenpunkt von 7° und ein Skalenpunkt von 9° auf der R-Seite nacheinander nach rechts angeordnet sind;
wobei die Bogenkante mit einer Skalenrille versehen ist, die jeweils den Skalenpunkt von 0°, den Skalenpunkt von 3°, den Skalenpunkt von 5°, den Skalenpunkt von 7° und den Skalenpunkt von 9° auf der L-Seite und den Skalenpunkt von 0°, den Skalenpunkt von 3°, den Skalenpunkt von 5°, den Skalenpunkt von 7° und den Skalenpunkt von 9° auf der R-Seite entspricht, und wobei das vordere Ende der Valguswinkel-Positionierungshülse mit einer konvexen Positionierungsspitze versehen ist, die in die Skalenrille eingeführt wird.

2. Positionierungsvorrichtung für die Osteotomie eines distalen Femurkondylusknochendefekts nach Anspruch 1, **dadurch gekennzeichnet, dass** das verschiebbare verriegelnde Positionierungsmodul auch zwei parallele optische Achsen umfasst, wobei das linke Ende und das rechte Ende einer Bodenplatte des verschiebbaren verriegelnden Positionierungsmodulkörpers jeweils mit einer der parallelen optischen Achsen verbunden sind, wobei die linke Seite und die rechte Seite des distalen Femur-Valguswinkel-Positionierungsmodulkörpers jeweils mit einem Positionierungsgleitloch versehen sind, und wobei die beiden parallelen optischen Achsen entsprechend in die beiden Positionierungsgleitlöcher eingesetzt sind.

3. Positionierungsvorrichtung für die Osteotomie eines distalen Femurkondylusknochendefekts nach Anspruch 1, **dadurch gekennzeichnet, dass** die linke Seite und die rechte Seite der Oberseite des distalen Femurknochendefekt-Osteotomiemoduls beide mit schrägen Nagellöchern zur Fixierung versehen sind und die Vorderseite der Oberseite des distalen Femurknochendefekt-Osteotomiemoduls mit Nagellöchern mit einstellbaren Positionen versehen ist.

4. Positionierungsvorrichtung für die Osteotomie eines distalen Femurkondylusknochendefekts nach Anspruch 1, **dadurch gekennzeichnet, dass** an der hinteren Endfläche des distalen Femurknochendefekt-Osteotomiemoduls ein Verbindungsloch angeordnet ist, an der vorderen Endfläche des verschiebbaren verriegelnden Positionierungsmodulkörpers eine Verbindungsstange angeordnet ist und die Verbindungsstange in das Verbindungsloch eingeführt ist.

5. Positionierungsvorrichtung für die Osteotomie eines distalen Femurkondylusknochendefekts nach Anspruch 1, **dadurch gekennzeichnet, dass** die Oberseite des verschiebbaren verriegelnden Positionierungsmodulkörpers mit zwei seitlichen Stützplatten versehen ist, wobei ein Federmontageloch auf einer Verbindungsfläche zwischen den beiden seitlichen Stützplatten angeordnet ist, wobei eine in das Federmontageloch eingesetzte Feder unterhalb des hinteren Endes des pressenden verriegelnden Hakens angeordnet ist, wobei der mittlere Teil des pressenden verriegelnden Hakens zwischen den beiden seitlichen Stützplatten durch einen Stützschaft abgestützt und positioniert ist, und wobei das vordere Ende des pressenden verriegelnden Hakens ein Haken ist, der lösbar in die Korrektur-Osteotomienuten eingehakt ist.

6. Positionierungsvorrichtung für die Osteotomie eines distalen Femurkondylusknochendefekts nach Anspruch 1, **dadurch gekennzeichnet, dass** der distale Femur-Valguswinkel-Positionierungsmodulkörper eine obere Seitenplatte und eine untere Seitenplatte umfasst, wobei ein Kipploch zwischen der oberen Seitenplatte und der unteren Seitenplatte angeordnet ist, wobei das vordere Ende des Alguswinkel-Kipphebels in das Kipploch eingeführt ist und mit der oberen Seitenplatte durch eine Befestigungsschraube fest verbunden, wobei die Valguswinkel-Einstellscheibe zwischen dem Valguswinkel-Kipphebel und der oberen Seitenplatte angeordnet ist, wobei eine Drehwelle ist am hinteren Ende auf der Mittellinie der oberen Seitenplatte angeordnet, und das vordere Ende der Alguswinkel-Einstellscheibe durch die rotierende Welle drehfest mit der oberen Seitenplatte verbundeni st.

7. Positionierungsvorrichtung für die Osteotomie eines distalen Femurkondylusknochendefekts nach Anspruch 1, **dadurch gekennzeichnet, dass** die Korrekturabmessungen der drei Korrektur-Osteotomienuten 0, 5 mm bzw. 10 mm betragen.

8. Positionierungsvorrichtung für die Osteotomie eines distalen Femurkondylusknochendefekts nach Anspruch 1, **dadurch gekennzeichnet, dass** der pressenden verriegelnden Haken mit mehreren konvexen Kanten versehen ist.

9. Positionierungsvorrichtung für die Osteotomie eines distalen Femurkondylusknochendefekts nach Anspruch 2, **dadurch gekennzeichnet, dass** das untere Ende einer der parallelen optischen Achsen mit einem Antiseparationsmechanismus versehen ist.

10. Positionierungsvorrichtung für die Osteotomie eines distalen Femurkondylusknochendefekts nach Anspruch 2, **dadurch gekennzeichnet, dass** eines der beiden Positionierungsgleitlöcher ein kreisförmiges Loch ist und das andere ein elliptisches Loch ist.

## Revendications

1. Un dispositif de positionnement d'ostéotomie du défaut du condyle fémoral distal, **caractérisé en ce qu'**il comprend un module d'ostéotomie du défaut fémoral distal, un module de positionneur coulissant et verrouillable et un module de positionnement d'angle valgus du fémur distal, dans laquelle une surface de montage correspondant à un condyle antérieur du fémur est disposée sur le côté avant du fond du module d'ostéotomie du défaut fémoral distal, et trois rainures de correction ostéotomie sont disposées parallèlement dans la direction antéropostérieur dans la côté arrière du haut du module d'ostéotomie du défaut fémoral distal;
Le module de positionneur coulissant et verrouillable est relié à l'arrière du module d'ostéotomie du défaut fémoral distal et comprend un corps de module de positionneur coulissant et verrouillable et un crochet de verrouillage à pression, la partie centrale du crochet de verrouillage à pression est reliée de manière rotative à la partie supérieure du corps du module de positionneur coulissant et verrouillable, un ressort est disposé entre la partie inférieure de l'extrémité arrière du crochet de verrouillage à pression et le corps du module de positionneur coulissant et verrouillable, et l'extrémité avant du crochet de verrouillage à pression est un crochet qui peut être accroché de manière amovible avec les rainures de correction ostéotomie;
Le module de positionnement d'angle valgus du fémur distal est disposé sous le module de positionneur coulissant et verrouillable, y compris un corps de module de positionnement d'angle valgus du fémur distal, une canule de positionnement d'angle valgus, un cadran d'angle valgus et un levier de bascule d'angle valgus; l'extrémité avant du levier de bascule d'angle valgus est insérée dans et fixée au corps du module de positionnement d'angle valgus du fémur distal, et l'extrémité arrière du levier de bascule d'angle valgus s'étend de l'arrière du corps du module de positionnement d'angle valgus du fémur distal et est gainée avec la canule de positionnement d'angle valgus, et la partie arrière de la canule de positionnement d'angle valgus est limitée par un écrou de fixation vissé avec le levier de bascule d'angle valgus;
Le cadran d'angle valgus est disposé au-dessus du levier de bascule d'angle valgus et relié de manière rotative au corps du module de positionneur d'angle valgus du fémur distal, l'extrémité du cadran d'angle valgus est un bord arqué, le centre du bord arqué est un point de numérotation 0°, l'axe de la vis de fixation, l'axe de l'arbre rotatif et le point de cadran 0° sont positionnés sur la même surface verticale, les points de numérotation à 3°, 5°, 7° et 9° sur le côté L sont disposés en séquence du point de numérotation à 0° vers la gauche, et les points de numérotation à 3°, 5°, 7° et 9° sur le côté R sont disposés en ordre vers la droite;
Le bord arqué est pourvu d'une rainure de cadran correspondant respectivement aux points de numérotation à 0°,3°, 5°, 7° et 9° du côté L et points de numérotation à 0°,3°, 5°, 7° et 9° du côté R, et l'extrémité avant de la canule de positionnement d'angle valgus est pourvue d'une pointe de positionnement convexe qui est insérée dans la rainure de cadran.

2. Dispositif de positionnement d'ostéotomie du défaut du condyle fémoral distal selon la revendication 1, **caractérisé en ce que** le module de positionneur coulissant et verrouillable comprend également deux axes optiques parallèles, l'extrémité gauche et droite de la plaque inférieure du corps du module de positionneur coulissant et verrouillable sont respectivement reliées à l'un des axes optiques parallèles, deux trous coulissant de positionnement sont disposés respectivement sur le côté gauche et le côté droite du corps du module de positionnement d'angle fémoral distal, et les deux axes optiques parallèles sont insérés dans les deux trous coulissant de positionnement correspondants.

3. Dispositif de positionnement d'ostéotomie du défaut du condyle fémoral distal selon la revendication 1, **caractérisé en ce que** le côté gauche et le côté droite de la partie supérieure du module d'ostéotomie du défaut fémoral distal sont tous pourvus de trous obliques pour la fixation, et le côté avant de la partie supérieure du module d'ostéotomie du défaut fémoral distal est pourvu de trous pour régler la position.

4. Dispositif de positionnement d'ostéotomie du défaut du condyle fémoral distal selon la revendication 1, **caractérisé en ce qu'**un trou de connexion est disposé sur la surface d'extrémité arrière du module d'ostéotomie du défaut fémoral distal, une tige de raccordement est disposée sur la surface d'extrémité avant du corps du module de positionneur coulissant et verrouillable, et la tige de raccordement est insérée dans le trou de connexion.

5. Dispositif de positionnement d'ostéotomie du défaut du condyle fémoral distal selon la revendication 1, **caractérisé en ce que** la partie supérieure du corps du module de positionneur coulissant et verrouillable est pourvu de deux plaques de support latérales, un trou de montage de ressort est disposé sur la surface de raccordement entre les deux plaques de support latérales, un ressort inséré dans le trou de montage de ressort est disposé sous l'extrémité arrière du crochet de verrouillage à pression, la partie centrale du crochet de verrouillage à pression est soutenue et positionnée entre les deux plaques de support latérales par l'intermédiaire d'un arbre de support, et l'extrémité avant du crochet de verrouillage à pression est un crochet qui est accroché de manière amovible avec la rainure de correction ostéotomie.

6. Dispositif de positionnement d'ostéotomie du défaut du condyle fémoral distal selon la revendication 1, **caractérisé en ce que** le corps du module de positionnement d'angle valgus du fémur distal comprend une plaque supérieure et une plaque inférieure, un trou de bascule est disposé entre la plaque supérieure et la plaque inférieure et l'extrémité avant, l'extrémité avant du levier de bascule d'angle valgus est insérée dans le trou de bascule est fixée avec la plaque supérieure par l'intermédiaire d'une vis de fixation, le cadran d'angle valgus est disposé entre le levier de bascule d'angle valgus et la plaque supérieure, un arbre rotatif est disposé à l'extrémité arrière sur la ligne centrale de la plaque supérieure, et l'extrémité avant du cadran d'angle valgus est reliée de manière rotative à la plaque supérieure par l'intermédiaire de l'arbre rotatif.

7. Dispositif de positionnement d'ostéotomie du défaut du condyle fémoral distal selon la revendication 1, **caractérisé en ce que** les dimensions correctrices des trois rainures de correction ostéotomie sont respectivement de 0.5mm et 10mm.

8. Dispositif de positionnement d'ostéotomie du défaut du condyle fémoral distal selon la revendication 1, **caractérisé en ce que** le crochet de verrouillage à pression comporte une pluralité de bords convexes.

9. Dispositif de positionnement d'ostéotomie du défaut du condyle fémoral distal selon la revendication 2, **caractérisé en ce que** l'extrémité inférieure d'un des axes optiques parallèles est pourvue d'un mécanisme anti-détachement.

10. Dispositif de positionnement d'ostéotomie du défaut du condyle fémoral distal selon la revendication 2, **caractérisé en ce que** l'un des deux trous coulissants de positionnement est un trou circulaire, et l'autre un trou elliptique.
